(19) Europäisches Patentamt
European Patent Office
Office européen des brevets

(11) **EP 4 644 468 A1**

(12) **EUROPEAN PATENT APPLICATION**
published in accordance with Art. 153(4) EPC

(43) Date of publication:
**05.11.2025 Bulletin 2025/45**

(21) Application number: **22969755.2**

(22) Date of filing: **30.12.2022**

(51) International Patent Classification (IPC):
*C08J 3/24* (2006.01)    *C08J 3/075* (2006.01)
*C08K 5/17* (2006.01)    *A61L 27/52* (2006.01)

(52) Cooperative Patent Classification (CPC):
**A61L 27/52; C08J 3/075; C08J 3/24; C08K 5/17**

(86) International application number:
**PCT/CN2022/143868**

(87) International publication number:
**WO 2024/138610 (04.07.2024 Gazette 2024/27)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicant: **Imeik Technology Development Co., Ltd.**
**Beijing 100022 (CN)**

(72) Inventors:
• **WEN, Huifang**
  **Beijing 100022 (CN)**
• **LI, Ruizhi**
  **Beijing 100022 (CN)**
• **ZHANG, Kun**
  **Beijing 100022 (CN)**
• **GU, Shiwei**
  **Beijing 100022 (CN)**

(74) Representative: **Novagraaf Technologies**
  **Bâtiment O2**
  **2, rue Sarah Bernhardt**
  **CS90017**
  **92665 Asnières-sur-Seine Cedex (FR)**

(54) **POLYAMINO ACID GEL MATERIAL, PREPARATION METHOD THEREFOR AND USE THEREOF**

(57)    Provided is a polyamino acid gel material. The material is prepared by performing a pre-crosslinking treatment on an endogenous polyamine and a polyamino acid under the action of an activator, then freezing and crosslinking same, then thawing same at room temperature, and cyclically repeating the freezing-thawing process 0-3 times so as to obtain a polyamino acid gel material, wherein the polyamino acid is a polyglutamic acid or polyaspartic acid, and the endogenous polyamine is spermine or spermidine.

FIG. 1

EP 4 644 468 A1

**Description**

**Technical Field**

The present disclosure relates to the technical field of biomedical materials, and specifically, to a polyamino acid gel material, a preparation method therefor and a use thereof.

**Background**

Cryogels are a class of hydrogels with controlled polymerization at temperatures below a freezing point of a solvent, with frozen solvent crystals acting as interconnecting porogenic agents. As the temperature decreases, a large number of ice crystal templates are formed in a system, and the templates are melted to obtain connected macroporous gels, of which most prominent feature is a connected macroporous structure. Such macroporous structure can accelerate the supply of nutrients and the removal of metabolites, and the interconnected macropores can better mimic the in vivo environment, facilitating inward growth of blood vessels. A usual method for introducing an interpenetrating macroporous structure in a hydrogel network includes the use of a porogenic agent that can damage a network structure, such as crystalline particles, immiscible solvents, air bubbles, etc., but these porogenic agents must be removed from the network. Although the hydrogel with hundreds of micrometer macropores and interpenetrating network structures can be synthesized by using the porogenic agent, the porogenic agent remaining in the hydrogel is not only potentially toxic when used as a biomaterial, but may also form a physical barrier to impede cell penetration. Therefore, the preparation of a cryogel with good biocompatibility, a non-toxic crosslinking agent, and a good mechanical property is essential.

Polyamino acid hydrogels have attracted much attention because of their excellent water absorption and retention similar to synthetic polymers, as well as their biocompatibility and biodegradability similar to natural polymers. The polyamino acid hydrogel mainly includes Polyglutamic Acid (PGA) and Polyaspartic Acid (PASP). The polyglutamic acid is also referred to as a natto gum or polyglutamic acid, which is a water-soluble, biodegradable, and non-toxic biopolymer. The polyglutamic acid has excellent characteristics of being high in molecular weight, easy to disperse, non-toxic, harmless, edible, etc., and is a new green polymer material. The polyglutamic acid is non-immunogenic, can be completely degraded in nature or in the human bodies, is safe and non-toxic for food, medicine or cosmetic use, and thus may be used for studies on tissue engineering, and the like, and is a new polymer material with high development potential. The main chain of the polyglutamic acid contains a high number of active free side chain carboxyl (-COOH). The polyglutamic acid has a unique polypeptide chain secondary structure and a chiral characteristic, and thus is easy to modify. Furthermore, the polyglutamic acid has stronger water absorption than that of hyaluronic acid, and also has good biocompatibility and degradability. Small molecule amino acids degraded by the polyglutamic acid do not accumulate in the body and do not cause toxic side effects, such that the polyglutamic acid has good biocompatibility.

The polyaspartic acid is a green biodegradable polymer material of great interest, which referred to as PASP and may be obtained by biological or chemical methods through dehydration and condensation. A chemical structure of the polyaspartic acid is similar to that of proteins in that both are bound by peptide bonds. The peptide bonds are easily degraded and broken by microorganisms, and polymer macromolecules are broken into small molecule chains, which are eventually decomposed into water, carbon dioxide, and nitrogen-containing compounds, such that the polyaspartic acid has good biodegradability, and is green and non-polluting to environments. The polyaspartic acid hydrogel (PASP hydrogel) is a material with a spatial network structure prepared by crosslinking molecular chains of linear PASP through a crosslinking agent. A main chain thereof contains a large number of hydrophilic carboxyl and peptide bonds, and side groups have ether bonds and hydroxyl, all of which can bind to water molecules. Furthermore, the PASP molecular chains are soft and easy to stretch, such that the polyaspartic acid has high water storage and water retention characteristics, can be elastically deformed based on external forces, and has good flexibility and moldability. Furthermore, as an amino acid polymer, a peptide main chain of the polyaspartic acid is similar to a structure of the proteins, such that the polyaspartic acid has the advantages of being good in biocompatibility, easy to absorb by organisms, etc. It was found, through research, that the PASP may be digested and absorbed by the action of proteolytic enzymes without showing antigenicity, and its metabolites are non-toxic. Therefore, the PASP hydrogel has a wide range of uses and vast market prospects in the fields of agriculture, horticulture, medicine and biology, etc., and thus becomes a current research hotspot.

From the current research status of polyglutamic acid or polyaspartic acid hydrogels at home and abroad, the hydrogels have made achievements in many fields, but there are still some defects in their properties such as strength, elasticity, toughness, antibacterial performance, and the like, which not only limits the development of the polyamino acid hydrogel but also has a great limitation to its biological function, such that the preparation of a polyamino acid cryogel with a high mechanical property is extremely important.

Endogenous polyamine refers to polyamine that can be produced during synthetic or metabolic processes in a human environment, and the main endogenous polyamine includes the spermine, the spermidine, and putrescine. Endogenous polyamine-spermidine is a natural small molecule that is widely found in living organisms, has anti-rejection and anti-inflammatory properties, promotes autophagy, and prolongs the lifespan of individuals. With the help of biomimetic design ideas, by applying the bioactive molecule spermidine to the fields of biomedicine, tissue engineering, etc., a new solution may be provided for the immune rejection response faced by artificial tissues or organs during transplantation, so as to achieve better physiological functions and therapeutic effects. Literature (Madeo et al., Science359,410,2018) reported that the endogenous polyamine, such as the spermidine, has specific physiological roles, including, but not limited to, circadian rhythm regulation, hypertension improvement, cardiovascular protection, Alzheimer's disease prevention, immunity enhancement, cancer resistance, and even aging prevention. The physiologically active effects of the spermidine are manifested in the following aspects: 1) kidney: tension reduction, and aging prevention; 2) heart: blood pressure lowering, and arteriosclerosis prevention; 3) brain: memory decline prevention, Alzheimer's disease resistance, neuroprotective effect; 4) bone: prevention of bone loss affected by ovariectomy; 5) skeletal muscle: increasing of the temperature of aged muscle, and muscle disease prevention; 6) whole organism: living organism lifespan prolonging; 7) immune system: improvement of immune activity after vaccination, improvement of cancer directed immunity, and fatal sepsis prevention; 8) liver: prevention of liver fibrosis and canceration, etc. Main mechanisms for the physiological activity of the spermidine are that, the spermidine is polycationic ($-NH_3^+$) fatty amine that exists in a multiprotonated form under physiological pH conditions and is highly biologically active, and nucleic acids containing acidic residues, phospholipids, acidic proteins, pectic polysaccharides containing carboxylates or sulfates, as well as neurotransmitters and hormones (e.g., dopamine, adrenaline, serotonin, thyroid hormones, etc.) with a similar structure, are all possible targets for spermidine binding.

Cross-linked polyglutamic acid or polyaspartic acid cryogels are of hydrophilic polymer network structures, have high water absorption, high mechanical strength, controlled degradation, and biocompatibility, and are ideal materials for the preparation of wound dressings and tissue engineering scaffolds. At present, there are few reports on the use of the endogenous polyamine such as the spermidine as the crosslinking agent for the polyamino acid-based cryogels, and there are virtually no reports on the freeze-crosslinking reaction conditions of the endogenous polyamine with the polyglutamic acid or polyaspartic acid-based cryogels and the performance of the cryogels, in view of which the present disclosure is proposed.

## Summary

The present disclosure is intended to provide a polyamino acid gel material. Spermine or spermidine in endogenous polyamine is selected as a crosslinking agent, a polyamino acid gel is prepared through pre-crosslinking and freeze-crosslinking, and various physical and chemical properties of the prepared gel material may be regulated and controlled by controlling a pre-crosslinking temperature and time, a degree of crosslinking, a concentration, a reaction temperature, a reaction time, a thawing temperature, a thawing time, a pH, and a type of an activating agent, etc.

The present disclosure provides the following technical solutions.

In an aspect, the present disclosure provides a polyamino acid gel material; the material is obtained by performing pre-crosslinking and freezing-thawing treatments on endogenous polyamine and polyamino acid under the action of an activating agent, where the crosslinking of the endogenous polyamine and the polyamino acid includes three-site crosslinking or four-site crosslinking.

Further, the endogenous polyamine includes spermidine (a triamino compound) and/or spermine (a tetraamino compound).

In the present disclosure, the spermine or spermidine of the endogenous polyamine is selected as a crosslinking agent. Through the pre-crosslinking treatment, an imino group in the spermidine or spermine may participate in a crosslinking reaction to form multi-site crosslinking such as three sites or four sites, so as to form a compact interpenetrating network structure, thereby increasing thermal stability of the formed amide bond-crosslinked polyamino acid gel, and reducing safe risks caused by residual chemical crosslinking agents. The use of endogenous diamine such as putrescine is avoided in the present disclosure. In an aspect, the diamine such as putrescine has high toxicity, and in another aspect, the endogenous diamine such as putrescine can only form double-site crosslinking, and does not have the possibility of realizing multi-site crosslinking.

Further, the polyamino acid is polyglutamic acid and/or polyaspartic acid.

Further, a pore diameter of the gel material is 80-760 μm, and includes, but is not limited to, 80 μm, 90 μm, 100 μm, 120 μm, 150 μm, 160 μm, 170 μm, 180 μm, 200 μm, 250 μm, 300 μm, 350 μm, 400 μm, 450 μm, 500 μm, 550 μm, 600 μm, 650 μm, 700 μm, 720 μm, 750 μm, or 760 μm.

And/or, the porosity of the gel material is more than 95%, preferably, more than 98%.

The present disclosure uses the endogenous polyamine as a crosslinking agent, and prepares a polyglutamic acid-based gel or polyaspartic acid-based gel through pre-crosslinking and freezing-thawing processes. The gel has a larger pore diameter and higher porosity, causing the cryogel to have wider application prospects.

Further, the activating agent includes one or more of water-soluble carbodiimide, a carbonium salt, and 4-(4,6-dimethoxy-1,3,5-triazin-2-yl)-4-methyl morpholinium chloride (DMTMM).

A second aspect of the present disclosure further provides a method for preparing a polyamino acid gel material. The preparation method includes the following steps.

Polyamino acid and endogenous polyamine are mixed to obtain a mixed solution, a pH of the mixed solution is regulated to 4.00-7.40, an activating agent is added, pre-crosslinking is performed for 5-60 minutes at 25-60 °C after stirring, then freeze-crosslinking is performed, a product after freeze-crosslinking is thawed, and then a freezing-thawing process is repeated for 0-3 times, so as to obtain a gel material.

Pre-crosslinking is required before freezing, so as to ensure that single-site crosslinking of the spermine or spermidine and the polyamino acid is completed before freezing, that is, 1 amino in the spermine or spermidine participates into the reaction. Through the single-site crosslinking, the ends of spermine or spermidine molecules crosslinked on a molecular chain of a polyamino acid substance fixed by ice crystals after freezing are fixed, such that a distance thereof between the spermine or spermidine molecules and the molecular chain of the polyamino acid substance is shortened, steric hindrance between the spermine or spermidine molecules and the imino group is reduced, the collision probability of the imino group and aminos on the other ends in the spermine or spermidine and carboxyl of the molecular chain of the polyamino acid substance is improved, reaction efficiency is improved, and the probability of occurrence of multi-site crosslinking (i.e., three-site or four-site crosslinking) is also improved, thereby forming the cryogel having a macropore interpenetrating network structure with high porosity, and realizing effective monitoring of spermidine or spermine residues.

If the pre-crosslinking treatment is not performed before freezing, there is a large distance between spermine or spermidine molecules and the molecular chain of the polyamino acid that has been fixed by the ice crystals after freezing, the imino group with low crosslinking reaction activity has an extremely-low collision probability with the carboxyl of the molecular chain of the polyamino acid substance, leading to a reduction in efficiency during the crosslinking process, such that multi-site crosslinking gel such as three-site or four-site with a stable structure cannot be formed, a stable interpenetrating network structure cannot be formed, and thus a good hole-making effect cannot be achieved.

Furthermore, if the pre-crosslinking reaction treatment is not within the above range, high or low pre-crosslinking efficiency thereof leads to low crosslinking efficiency in the subsequent freezing process, making it difficult to form the multi-site crosslink, such that a network structure of the obtained gel is not compact enough, and stability is relatively poor.

Preferably, a temperature for freeze-crosslinking is -80 °C to -10 °C, and a time is 2-48 h; and a temperature for thawing is 10-60 °C, and a thawing time is 0.5-8h.

Preferably, the endogenous polyamine includes spermine (a tetraamino compound) and/or spermidine (a triamino compound).

Preferably, the activating agent includes one or more of water-soluble carbodiimide, a carbonium salt, and 4-(4,6-dimethoxy-1,3,5-triazin-2-yl)-4-methyl morpholinium chloride.

Preferably, the polyamino acid includes polyglutamic acid and/or polyaspartic acid.

And/or, a solvent of the mixed solution is selected from the mixing of one or more of water, soluble alcohol, soluble ketone, DMF, DMA, and DMSO; and preferably, the solvent is water.

Preferably, the soluble alcohol includes the mixing of one or more of methanol, ethanol, propanol, and isopropanol.

Preferably, the soluble ketone is acetone.

Further, a molecular weight of the polyamino acid is within a conventional range.

In some embodiments of the present disclosure, the solvent of the mixed solution is water.

In some embodiments of the present disclosure, the pH of the mixed solution = 4.00-7.40. For example, the pH includes, but is not limited to, 4.00, 4.10, 4.15, 4.20, 4.25, 4.30, 4.35, 4.40, 4.45, 4.50, 4.60, 4.70, 4.80, 4.90, 5.00, 5.10, 5.20, 5.30, 5.40, 5.50, 5.60, 5.70, 5.80, 5.90, 6.00, 6.10, 6.20, 6.30, 6.32, 6.35, 6.38, 6.40, 6.42, 6.45, 6.50, 6.55, 6.58, 6.60, 6.62, 6.65, 6.68, 6.70, 6.73, 6.75, 6.80, 6.85, 6.90, 6.95, 7.00, 7.05, 7.10, 7.20, 7.30, or 7.40.

Preferably, the pH of the mixed solution is regulated to 4.00-7.40. By controlling the pH of the mixed solution within the range, the pore diameter and porosity of the obtained gel may be regulated and controlled relatively stably. If the pH of the solution is higher than 7.4, a solution system is alkaline, easily leading to hydrolysis of active ester generated by polyglutamic acid and polyaspartic acid and a catalyst, seriously reducing the reaction efficiency of the polyamino acid and the endogenous polyamine, and thus affecting the crosslinking efficiency. If the pH value of the solution is lower than 4, the solution system is acidic, easily leading to hydrolysis of the activating agent, and thus making it difficult to complete the crosslinking reaction effectively.

Preferably, when the pH of the mixed solution is controlled to be 6.3-7.4, the pore diameter of the obtained gel is between 80-154 $\mu$m, the porosity is about 95%, and the pH includes, but is not limited to, 6.30, 6.35, 6.40, 6.45, 6.50, 6.55, 6.60, 6.65, 6.70, 6.75, 6.80, 6.85, 6.88, 6.90, 7.00, 7.10, 7.15, 7.20, 7.25, 7.30, 7.35, or 7.40.

When the pH of the mixed solution is controlled to be 5.40-6.20, the pore diameter of the obtained gel is between 155-419 $\mu$m, the porosity is between 95-96%, and the pH includes, but is not limited to, 5.40, 5.45, 5.50, 5.55, 5.58, 5.60, 5.65, 5.70, 5.75, 5.80, 5.85, 5.90, 5.95, 6.00, 6.05, 6.10, 6.15, or 6.20.

More preferably, when the pH of the mixed solution is controlled to be 4.00-5.30, the pore diameter of the obtained gel is between 420-760 μm, the porosity is between 97-98%, and the pH includes, but is not limited to, 4.00, 4.05, 4.10, 4.15, 4.20, 4.25, 4.30, 4.35, 4.40, 4.45, 4.50, 4.55, 4.60, 4.65, 4.70, 4.75, 4.80, 4.85, 4.90, 4.95, 5.00, 5.10, 5.15, 5.20, 5.25, 5.30, or 5.30.

In some embodiments of the present disclosure, a freezing temperature is -80 °C-10 °C, and includes, but is not limited to, -80 °C, -79 °C, -78 °C, -77 °C, -76 °C, -75 °C, -74 °C, -73 °C, -72 °C, -71 °C, -70 °C, -69 °C, -68 °C, -67 °C, -66 °C, -65 °C, -64 °C, -63 °C, -62 °C, -61 °C, -60 °C, -59 °C, -58 °C, -57 °C, -56 °C, -55 °C, -54 °C, -53 °C, -52 °C, -51 °C, -50 °C, -49 °C, -48 °C, -47 °C, -46 °C, -45 °C, -44 °C, -43 °C, -42 °C, -41 °C, -40 °C, -39 °C, -38 °C, -37 °C, -36 °C, -35 °C, -34 °C, -33 °C, -32 °C, -31 °C, -30 °C, -29 °C, -28 °C, -27 °C, -26 °C, -25 °C, -24 °C, -23 °C, -22 °C, -21 °C, -20 °C, -19 °C, -18 °C, -17 °C, -16 °C, -15 °C, -14 °C, -13 °C, -12 °C, -11 °C, or -10 °C.

Preferably, a freezing time is 2-48 h, and includes, but is not limited to, 2 h, 3 h, 4 h, 5 h, 6 h, 7 h, 8 h, 9 h, 10 h, 11 h, 12 h, 13 h, 14 h, 15 h, 16 h, 17 h, 18 h, 19 h, 20 h, 21 h, 22 h, 23 h, 24 h, 25 h, 26 h, 27 h, 28 h, 29 h, 30 h, 31 h, 32 h, 33 h, 34 h, 35 h, 36 h, 37 h, 38 h, 39 h, 40 h, 41 h, 42 h, 43 h, 44 h, 45 h, 46 h, 47 h, or 48 h.

Preferably, a thawing temperature is 10-60 °C, and includes, but is not limited to, 10 °C, 11 °C, 12 °C, 13 °C, 14 °C, 15 °C, 16 °C, 17 °C, 18 °C, 19 °C, 20 °C, 21 °C, 22 °C, 23 °C, 24 °C, 25 °C, 26 °C, 27 °C, 28 °C, 29 °C, 30 °C, 31 °C, 32 °C, 33 °C, 34 °C, 35 °C, 36 °C, 37 °C, 38 °C, 39 °C, 40 °C, 41 °C, 42 °C, 43 °C, 44 °C, 45 °C, 46 °C, 47 °C, 48 °C, 49 °C, 50 °C, 51 °C, 52 °C, 53 °C, 54 °C, 55 °C, 56 °C, 57 °C, 58 °C, 59 °C, or 60 °C.

Preferably, a thawing time is 0.5-8 h. The thawing time includes, but is not limited to, 0.5 h, 1 h, 2 h, 3 h, 3.5 h, 4 h, 4.5 h, 5 h, 5.5 h, or 6 h.

Further, the water-soluble carbodiimide activating agent includes 1-ethyl-3-(3-dimethylaminopropyl)carbodiimide (EDC), 1-(3-dimethylaminopropyl)-3-ethyl-carbodiimide, 1-cyclohexyl-3-(2-morpholinoethyl)carbodiimide, 1,3-bis[di(methoxymethyl)methyl]carbodiimide, or salts thereof, as well as a mixture of one or more of the above.

The carbonium salt includes a mixture of one or more of O-(7-azabenzotriazole-1-yl)-bis(dimethylamino)carbonium hexafluorophosphate (HATU), O-(benzotriazol-1-yl)-bis(dimethylamino)carbonium hexafluorophosphate (HBTU), O-(5-chlorobenzotriazol-1-yl)-bis(dimethylamino)carbonium hexafluorophosphate (HCTU), O-(benzotriazol-1-yl)-bis(dimethylamino)carbonium tetrafluoroborate (TBTU), O-(N-succinimidyl)-bis(dimethylamino)carbonium tetrafluoroborate (TSTU), and 2-(5-norborene-2,3-dicarboximido)-1,1,3,3-tetramethyluronium tetrafluoroborate (TNTU).

In some embodiments of the present disclosure, when the activating agent is the water-soluble carbodiimide activating agent, the method further includes: an auxiliary agent is added to the mixed solution to cause the activating agent to be used in combination with the auxiliary agent, so as to improve crosslinking reaction efficiency.

The auxiliary agent includes any one or more of N-hydroxysuccinimide (NHS), sulfonated N-hydroxysuccinimide (S-NHS), tert-butanol, and 1-hydroxybenzotriazole (HOBt).

More preferably, the auxiliary agent is added in an amount of 10-50% of the mass of the carbodiimide, including, but not limited to, 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, or 50%, preferably, the auxiliary agent is added in an amount of 15-50% of the mass of the carbodiimide.

Further, when the endogenous polyamine is the spermine, the spermine is added in an amount of 1.0-62% of a molar weight of a polyamino acid structure unit, including, but not limited to, 1.0%, 1.5%, 2.2%, 2.5%, 3%, 4%, 5%, 6%, 7%, 8%, 9%, 10%, 13%, 15%, 16%, 17%, 18%, 19%, 20%, 21%, 22%, 23%, 24%, 25%, 26%, 27%, 28%, 29%, 30%, 31%, 32%, 32%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, and 62%, preferably, 9-18%.

When the endogenous polyamine is the spermidine, the spermidine is added in an amount of 0.5-52% of a molar weight of a polyamino acid structure unit, including, but not limited to, 0.5%, 0.8%, 1.0%, 1.5%, 2%, 2.5%, 3%, 5%, 6%, 7%, 8%, 10%, 11%, 12%, 13%, 14%, 15%, 16%, 17%, 18%, 19%, 20%, 21%, 22%, 23%, 24%, 25%, 26%, 27%, 28%, 29%, 30%, 34%, 38%, 40%, 41%, 42%, 43%, 44%, 45%, 46%, 47%, 48%, 49%, 50%, 51%, and 52%, preferably, 9-17%.

A mass concentration of the polyamino acid in the mixed solution is 10-160 mg/mL, and includes, but is not limited to, 10, 20, 30, 40, 50, 55, 60, 70, 75, 80, 90, 100, 110, 120, 130, 140, 150, or 160 mg/mL.

The activating agent is added in an amount of 0.5-550% of the molar weight of the polyamino acid structure unit, preferably 50-500%, and more preferably, 50-300%.

Further, the method further includes: the thawed polyamino acid cryogel is added to a phosphate buffer with a concentration of 10-25 mg/mL, and moist heat sterilization is performed to obtain a gel material.

Preferably, the pH of the phosphate buffer is 7.0.

Preferably, a temperature for moist heat sterilization is 100-130 °C, preferably 121 °C; and a time for moist heat sterilization is 10-30 min, preferably 15 min.

Further, the present disclosure provides the gel material prepared by the foregoing method.

A third aspect of the present disclosure further provides an application of the gel material of the first aspect or a gel material prepared by the method of the second aspect in preparation of medical, beauty, and health care products.

The medical and beauty products include: soft tissue filling materials, cartilage repair materials, tissue implant materials, coatings of biomaterial implants, tissue engineering scaffold materials, drug sustained-release media, drug targeting carriers, wound dressings, etc.

Preferably, the coatings of biomaterial implants include a breast implant, a catheter, a cannula, a bone repair, a cartilage substitute, a micropump and other drug delivery apparatuses, an artificial organ and blood vessel, and a coating of a mesh for tissue reinforcement.

Preferably, the soft tissue filling materials include filling materials for the face, neck, head, ears, breasts, or joints, or a joint lubricant for medical use.

More preferably, the filling material is an intradermal or subcutaneous implantable material used for improving the quality of the skin or for filling in wrinkles or for restoring volume to the face or body.

Preferably, a wound healing drug is the wound dressing.

Preferably, the medical and beauty products further include a therapeutic active agent.

Preferably, the therapeutic active agent includes a chemotherapeutant or a biologically active factor.

More preferably, the active factor includes an anti-inflammatory agent, an antibiotic, a painkiller, an anesthetic, a wound healing promoter, a cell growth inhibitor, an immunostimulant, an immunosuppressant, and an antiviral drug.

The health care product includes capsules, tablets, health drinks, and the like.

The present disclosure has the following beneficial effects.

(1) Before the obtained gel of the present disclosure is frozen, the pre-crosslinking treatment is performed to control the reaction process of the gel. By completing the single-site crosslinking of the spermine or spermidine and the polyamino acid substance, that is, 1 amino in the spermine or spermidine participates into the reaction, the ends of the spermine or spermidine molecules crosslinked on the molecular chain of the polyamino acid substance fixed by the ice crystals after freezing are fixed, such that a distance thereof between the spermine or spermidine molecules and a polymer chain of the polyamino acid substance is shortened, steric hindrance between the spermine or spermidine molecules and the imino group is reduced, the collision probability of the imino group and aminos on the other ends in the spermine or spermidine and carboxyl of the molecular chain of the polyamino acid substance is improved, the reaction efficiency is improved, and the probability of occurrence of multi-site crosslinking is also improved, thereby forming the gel having a continuous interpenetrating macroporous structure with the porosity up to more than 95%. Furthermore, by controlling the pre-crosslinking reaction process and reaction conditions, the overall reaction efficiency of the cryogel may reach up to more than 90%, such that the amount of spermine or spermidine residues is greatly reduced, which is of great significance to the safety of clinical use of the product.

(2) In the present disclosure, while the pre-crosslinking reaction process is controlled, by regulating the pH value of the reaction system, a porous gel having different pore diameter gradients is obtained through preparation. When the pH is 6.3-7.4, the pore diameter of the obtained gel is 80-154 $\mu$m; when the pH is 5.40-6.20, the pore diameter of the obtained gel is 155-419 $\mu$m; and when the pH is 4.00-5.30, the pore diameter of the obtained gel is 420-760 $\mu$m.

(3) The present disclosure creatively selects the endogenous polyamine and the polyamino acid for pre-crosslinking, and undergoes the crosslinking reaction under the freezing-thawing conditions so as to generate the gel having a continuous interpenetrating porous structure. Such unique structure facilitates the exchange of substances and promotes cellular proliferation and adhesion, has high water absorption. The entire process is mild and controllable, and is high in yield. The present disclosure has potential research values in the field of biomaterials and tissue engineering and wide application prospects.

**Brief Description of the Drawings**

In order to more clearly illustrate the specific embodiments of the present disclosure or the technical solutions in the related art, the drawings used in the description of the specific embodiments or the related art will be briefly described below. It is apparent that the drawings in the following descriptions are some embodiments of the present disclosure. Other drawings can be obtained from those skilled in the art according to these drawings without any creative work.

In order to more clearly illustrate the specific embodiments of the present disclosure or the technical solutions in the related art, the drawings used in the description of the specific embodiments or the related art will be briefly described below. It is apparent that the drawings in the following descriptions are some embodiments of the present disclosure. Other drawings can be obtained from those skilled in the art according to these drawings without any creative work.

FIG. 1(a) shows an SEM test diagram of a gel C6 according to the present disclosure.

FIG. 1(b) shows a partial enlarged view of FIG. 1(a).

FIG. 2(a) shows an SEM test diagram of a gel B1 according to the present disclosure.

FIG. 2(b) shows a partial enlarged view of FIG. 2(a).

FIG. 3(a) shows an SEM test diagram of a gel B2 according to the present disclosure.

FIG. 3(b) shows a partial enlarged view of FIG. 3(a).

FIG. 4(a) shows an SEM test diagram of a gel B3 according to the present disclosure.

FIG. 4(b) shows a partial enlarged view of FIG. 4(a).

FIG. 5(a) shows an SEM test diagram of a gel B4 according to the present disclosure.

FIG. 5(b) shows a partial enlarged view of FIG. 5(a).

FIG. 6 shows a porosity test diagram of gels C1-C7 and B1-B4 according to the present disclosure.

FIG. 7 shows a swelling rate test result of gels C1-C7 and B1-B4 according to the present disclosure.

FIG. 8 shows a degradation curve of gels C1-C7 according to the present disclosure.

FIG. 9 shows a stress-strain curve of gels C6 and B1 according to the present disclosure.

FIG. 10 shows a test result of an impact of gels C1-C7 on cell proliferation according to the present disclosure.

**Detailed Description of the Embodiments**

Unless otherwise defined, all scientific and technical terms used in the present disclosure have the same meaning as is commonly understood by those skilled in the art to which the present disclosure relates.

In the present disclosure, the term "crosslinking" refers to a process in which a chemically reactive polymer with a linear structure is chemically converted into a polymer with a three-dimensional mesh (body) structure, which is often used for polymer modification.

In the present disclosure, the term "crosslinking agent" refers to a reagent that can generate a chemical bond between linear molecules, causing the linear molecules to be connected to each other to form a mesh structure, and is used to improve the strength of a polymer material.

In the present disclosure, the term "multi-site crosslinking" refers to three-site crosslinking or four-site crosslinking, rather than double-site crosslinking, where the three-site crosslinking specifically refers to spermidine as a crosslinking agent, and the four-site crosslinking specifically refers to spermine as the crosslinking agent.

In the present disclosure, the term "mixed solution" refers to a solution that is obtained by mixing a solvent-dissolved uncrosslinked polyamino acid substance with dissolved endogenous polyamine.

In the present disclosure, the term "room temperature" refers to 25 °C±5 °C.

The technical solutions of the present disclosure will be clearly and completely described below with reference to the embodiments. It is apparent that the described embodiments are a part of the embodiments of the present disclosure. Based on the embodiments in the present disclosure, all other embodiments obtained by those of ordinary skill in the art without creative work shall fall within the protection scope of the present disclosure.

Example 1: Preparation of gel C1 of DMTMM-catalyzed four-site crosslinking of spermine and polyglutamic acid

0.34 g of spermine (a mole number of 1.6 mmol) was weighed and placed in a beaker, then 75 mL of purified water was added, after the mixture was completely dissolved, 4.0 g of polyglutamic acid (a molecular weight of 1500 KDa, containing 27 mmol of a polyglutamic acid repeat structure unit) was weighed, and a content of the spermine accounted for 8.5% of the mass of the polyglutamic acid (i.e., accounting for 5.9% of a mole number of the polyglutamic acid repeat structure unit). A pH value of a polyglutamic acid solution was regulated to about 4.0 by using a 2 mol/L hydrochloric acid solution and a 2 mol/L sodium hydroxide solution, a DMTMM solution (1.81 g of DMTMM, and 5 mL of deionized water) was added, and the mixture was uniformly stirred, sealed, placed at 25 °C, subjected to pre-crosslinking for 60 minutes, then transferred to -80 °C to undergo a reaction for 16 h, and thawed for 8 h at 25 °C, so as to obtain a crude cryogel product. Then purified water was added, suction filtration was performed after crushing the gel with a homogenizer, and after 6 times of washing, an obtained filter cake was freeze-dried. 1 g of the freeze-dried gel was weighed, 50 mL of a phosphate buffer with a concentration of 20 mg/mL and the pH being 7.0 was added, and after the gel was completely swelled, the gel was filled in a 1 mL or 2 mL prefilled syringe, and moist heat sterilization was performed for 15 min at 121 °C, so as to obtain a final product gel C1.

Example 2: Preparation of gel C2 of EDC-catalyzed three-site crosslinking of spermidine and polyglutamic acid

0.32 g of spermidine (a mole number of 2.2 mmol) was weighed and placed in a beaker, then 52.14 mL of purified water was added, after the mixture was completely dissolved, 4.0 g of polyglutamic acid (a molecular weight of 1500 KDa, containing 27 mmol of a polyglutamic acid repeat structure unit) was weighed, and a content of the spermidine accounted for 8% of the mass of the polyglutamic acid (i.e., accounting for 8.1% of a mole number of the polyglutamic acid repeat structure unit). A pH value of a polyglutamic acid solution was regulated to about 5.3 by using a 2 mol/L hydrochloric acid solution and a 2 mol/L sodium hydroxide solution, an aqueous solution of EDC and NHS (1.25 g of EDC, 0.25 g of NHS, and 5 mL of deionized water) was added, and the mixture was uniformly stirred, sealed, placed at 40 °C, subjected to pre-crosslinking for 20 minutes, then transferred to -60 °C to undergo a reaction for 8 h, thawed for 4 h at 25 °C, then transferred to -20 °C for freezing for 16 h, and finally thawed for 3 h at 40 °C, so as to obtain a crude cryogel product. Then purified water was added, suction filtration was performed after crushing the gel with a homogenizer, and after 6 times of washing, an obtained filter cake was freeze-dried. 1 g of the freeze-dried gel was weighed, 50 mL of a phosphate buffer with a concentration of 20 mg/mL and the pH being 7.0 was added, and after the gel was completely swelled, the gel was filled in a 1 mL or 2 mL prefilled syringe, and moist heat sterilization was performed for 15 min at 121 °C, so as to obtain a final product gel C2.

Example 3: Preparation of gel C3 of HATU-catalyzed four-site crosslinking of spermine and polyglutamic acid

0.50 g of spermine (a mole number of 2.4 mmol) was weighed and placed in a beaker, then 30 mL of purified water was added, after the mixture was completely dissolved, 4.0 g of polyglutamic acid (a molecular weight of 1500 KDa, containing 27 mmol of a polyglutamic acid repeat structure unit) was weighed, and a content of the spermine accounted for 12.5% of the mass of the polyglutamic acid (i.e., accounting for 8.9% of a mole number of the polyglutamic acid repeat structure unit). A pH value of a polyglutamic acid solution was regulated to about 6.0 by using a 2 mol/L hydrochloric acid solution and a 2 mol/L sodium hydroxide solution, an aqueous solution of HATU (46.56 g of HATU, and 10 mL of deionized water) was added, and the mixture was uniformly stirred, sealed, placed at 30 °C, subjected to pre-crosslinking for 40 minutes, then transferred to -40 °C to undergo a reaction for 24 h, and finally thawed for 6 h at 30 °C, so as to obtain a crude cryogel product. Then purified water was added, suction filtration was performed after crushing the gel with a homogenizer, and after 6 times of washing, an obtained filter cake was freeze-dried. 1 g of the freeze-dried gel was weighed, 50 mL of a phosphate buffer with a concentration of 20 mg/mL and the pH being 7.0 was added, and after the gel was completely swelled, the gel was filled in a 1 mL or 2 mL prefilled syringe, and moist heat sterilization was performed for 15 min at 121 °C, so as to obtain a final product gel C3.

Example 4: Preparation of gel C4 of HATU-catalyzed three-site crosslinking of spermidine and polyglutamic acid

0.39 g of spermidine (a mole number of 2.7 mmol) was weighed and placed in a beaker, then 29.78 mL of purified water was added, after the mixture was completely dissolved, 4.0 g of polyglutamic acid (a molecular weight of 1500 KDa, containing 27 mmol of a polyglutamic acid repeat structure unit) was weighed, and a content of the spermidine accounted for 9.8% of the mass of the polyglutamic acid (i.e., accounting for 10.0% of a mole number of the polyglutamic acid repeat structure unit). A pH value of a polyglutamic acid solution was regulated to about 6.2 by using a 2 mol/L hydrochloric acid solution and a 2 mol/L sodium hydroxide solution, an aqueous solution of HATU (4.14 g of HATU, and 5 mL of deionized water) was added, and the mixture was uniformly stirred, sealed, placed at 35 °C, subjected to pre-crosslinking for 30 minutes, then transferred to -30 °C to undergo a reaction for 48 h, and finally thawed for 4 h at 35 °C, so as to obtain a crude cryogel product. Then purified water was added, suction filtration was performed after crushing the gel with a homogenizer, and after 6 times of washing, an obtained filter cake was freeze-dried. 1 g of the freeze-dried gel was weighed, 50 mL of a phosphate buffer with a concentration of 20 mg/mL and the pH being 7.0 was added, and after the gel was completely swelled, the gel was filled in a 1 mL or 2 mL prefilled syringe, and moist heat sterilization was performed for 15 min at 121 °C, so as to obtain a final product gel C4.

Example 5: Preparation of gel C5 of EDC-catalyzed four-site crosslinking of spermine and polyglutamic acid

0.62 g of spermine (a mole number of 3.0 mmol) was weighed and placed in a beaker, then 23.57 mL of purified water was added, after the mixture was completely dissolved, 4.0 g of polyglutamic acid (a molecular weight of 1500 KDa, containing 27 mmol of a polyglutamic acid repeat structure unit) was weighed, and a content of the spermine accounted for 15.5% of the mass of the polyglutamic acid (i.e., accounting for 11.1 % of a mole number of the polyglutamic acid repeat structure unit). A pH value of a polyglutamic acid solution was regulated to about 5.0 by using a 2 mol/L hydrochloric acid solution and a 2 mol/L sodium hydroxide solution, an aqueous solution of EDC and NHS (2.30 g of EDC, 0.46g g of NHS, and 5 mL of deionized water) was added, and the mixture was uniformly stirred, sealed, placed at 45 °C, subjected to pre-crosslinking for 15 minutes, then transferred to -60 °C to undergo a reaction for 8 h, thawed for 4 h at 25 °C, then transferred to -20 °C for freezing for 16 h, and finally thawed for 2 h at 45 °C, so as to obtain a crude cryogel product. Then purified water was added, suction filtration was performed after crushing the gel with a homogenizer, and after 6 times of washing, an obtained filter cake was freeze-dried. 1 g of the freeze-dried gel was weighed, 50 mL of a phosphate buffer with a concentration of 20 mg/mL and the pH being 7.0 was added, and after the gel was completely swelled, the gel was filled in a 1 mL or 2 mL prefilled syringe, and moist heat sterilization was performed for 15 min at 121 °C, so as to obtain a final product gel C5.

Example 6: Preparation of gel C6 of DMTMM-catalyzed four-site crosslinking of spermine and polyglutamic acid

0.84 g of spermine (a mole number of 4.1 mmol) was weighed and placed in a beaker, then 23.77 mL of purified water was added, after the mixture was completely dissolved, 4.0 g of polyglutamic acid (a molecular weight of 1500 KDa, containing 27 mmol of a polyglutamic acid repeat structure unit) was weighed, and a content of the spermine accounted for 21.00% of the mass of the polyglutamic acid (i.e., accounting for 15.2% of a mole number of the polyglutamic acid repeat structure unit). A pH value of a polyglutamic acid solution was regulated to about 5.5 by using a 2 mol/L hydrochloric acid solution and a 2 mol/L sodium hydroxide solution, an aqueous solution of DMTMM and NHS (11.29 g of DMTMM, and 7 mL of deionized water) was added, and the mixture was uniformly stirred, sealed, placed at 54 °C, subjected to pre-crosslinking for 10 minutes, then transferred to -50 °C to undergo a reaction for 4 h, thawed for 4 h at 25 °C, then transferred to -10 °C for freezing for 24 h, and finally thawed for 1 h at 54 °C, so as to obtain a crude cryogel product. Then purified water was added, suction filtration was performed after crushing the gel with a homogenizer, and after 6 times of washing, an obtained filter cake was freeze-dried. 1 g of the freeze-dried gel was weighed, 50 mL of a phosphate buffer with a concentration of 20 mg/mL and the pH being 7.0 was added, and after the gel was completely swelled, the gel was filled in a 1 mL or 2 mL prefilled syringe, and moist heat sterilization was performed for 15 min at 121 °C, so as to obtain a final product gel C6.

Example 7: Preparation of gel C7 of EDC-catalyzed three-site crosslinking of spermidine and polyaspartic acid

0.87 g of spermidine (a mole number of 6.0 mmol) was weighed and placed in a beaker, then 28.33 mL of purified water was added, after the mixture was completely dissolved, 4.0 g of polyaspartic acid (a molecular weight of 4000 KDa, containing 30 mmol of a polyaspartic acid repeat structure unit) was weighed, and in this case, a content of the spermidine accounted for 21.75% of the mass of the polyaspartic acid (i.e., accounting for 20.0% of a mole number of the polyaspartic acid repeat structure unit). A pH value of a polyaspartic acid solution was regulated to about 7.4 by using a 2 mol/L hydrochloric acid solution and a 2 mol/L sodium hydroxide solution, an aqueous solution of EDC (11.52 g of EDC, 2.3 g of NHS, and 5 mL of deionized water) was added, and the mixture was uniformly stirred, sealed, placed at 60 °C, subjected to pre-crosslinking for 8 minutes, then transferred to -65 °C to undergo a reaction for 2 h, thawed for 4 h at 25 °C, then transferred to -30 °C for freezing for 48 h, and finally thawed for 0.5 h at 60 °C, so as to obtain a crude cryogel product. Then purified water was added, suction filtration was performed after crushing the gel with a homogenizer, and after 6 times of washing, an obtained filter cake was freeze-dried. 1 g of the freeze-dried gel was weighed, 50 mL of a phosphate buffer with a concentration of 20 mg/mL and the pH being 7.0 was added, and after the gel was completely swelled, the gel was filled in a 1 mL or 2 mL prefilled syringe, and moist heat sterilization was performed for 15 min at 121 °C, so as to obtain a final product gel C7.

Comparative example 1: Preparation of gel B1 of DMTMM-catalyzed double-site crosslinking of spermine and polyglutamic acid

The low temperature freeze-thaw crosslinking process was the same as that in Example 6, only the pre-crosslinking process of "placing at 54 °C for pre-crosslinking for 10 minutes" was omitted, and a final product cryogel B1 was obtained.

Comparative example 2: Preparation of gel B2 of EDC-catalyzed double-site crosslinking of spermine and polyaspartic acid

The low temperature freeze-thaw crosslinking process was the same as that in Example 7, only the pre-crosslinking process of "placing at 60 °C for pre-crosslinking for 8 minutes" was omitted, and a final product cryogel B2 was obtained.

Comparative example 3: Preparation of gel B3 of DMTMM-catalyzed double-site crosslinking of spermine and polyglutamic acid

The low temperature freeze-thaw crosslinking process was the same as that in Example 6, only the operation of uniformly stirring the mixture, sealing same, and then "placing same at 70 °C for pre-crosslinking for 5 minutes" was performed, and a final product gel B3 was obtained.

Comparative example 4: Preparation of gel B4 of DMTMM-catalyzed double-site crosslinking of spermine and polyglutamic acid

The low temperature freeze-thaw crosslinking process was the same as that in Example 6, only the operation of uniformly stirring the mixture, sealing same, and then "placing same at 15 °C for pre-crosslinking for 65 minutes" was performed, and a final product gel B4 was obtained.

Test example 1: Determination of rheological property of gel

A rheological property test was performed on the gels C1-C7 and B1-B4 prepared in Examples 1-7 and Comparative examples 1-4. 2 mL of each gel before and after sterilization was taken and tested by using a rotational rheometer. Parameters of the rheometer included: operation gap: 1000 mm; loading gap: 45000 mm; operating temperature: 37 °C; deformation quantity: 1%; frequency: 0.9 Hz; and operating time: 60 s. Rheological data for each gel was shown in Table 2; and G' (elastic modulus) loss rate was calculated as follows.

$$G' \text{ loss rate} = (G' \text{ before sterilization} - G' \text{ after sterilization})/G' \text{ before sterilization}$$

Elastic modulus test results of the gels obtained in Examples 1-7 and Comparative examples 1-4 were shown in Table 1. G' of the gels C1-C7 obtained through the pre-crosslinking and freezing-thawing crosslinking processes after sterilization was above 3500 Pa, and the G' loss rates were all less than 3%, which might because the polyamino acid gel underwent three-site crosslinking or four-site crosslinking during the crosslinking reaction, thereby forming an interpenetrating network structure, such that the structure was compact, highly stable, and resistant to moist heat sterilization, and had a low G' loss rate. By comparing the data in Examples 6-7 with that in Comparative examples 1-2, it might be found that, the G' of the C6 after sterilization was 11.9 times of the G' of the B1 after sterilization, the G' of the C7 after sterilization was 11.4 times of the G' of the B2 after sterilization, and the G' loss rates of the C6 and C7 both were less than 2%, which were much less than the loss rate of the B1 (56.22%) and the B2 (56.67%). This might because the gels B1 and B2 were not subjected to the pre-crosslinking treatment, an imino group in the spermine barely participated in the crosslinking reaction, such that the crosslinking efficiency was reduced, leading to the crosslinking formed being the double-side crosslinking, the network structure was relatively loose, which was not possible to form a stable and compact network interpenetrating structure, resulting in a reduction in the stability of the gel and a high G' loss rate. The above further indicated that the multi-site crosslinking such as three-site or four-site might be formed through the pre-crosslinking treatment, thereby improving the stability of the gel. The elastic modulus G' and its loss rates in Comparative examples 3-4 after sterilization were also significantly higher than those in Example 6, which might because the pre-crosslinking treatment was not performed under appropriate conditions in Comparative examples 3-4, leading to the low crosslinking efficiency in the subsequent freezing process, it was difficult to form the multi-site crosslinking such as three-site or four-site in the obtained gel, as a result, a compact interpenetrating network structure was not formed, the stability was poor, and thus the loss rate of the elastic modulus G' was relatively high.

Table 1 Rheological data of gel

| Examples | Gel name | Before sterilization | After sterilization | G' loss rate |
|---|---|---|---|---|
| | | G' (Pa) | G' (Pa) | |
| Example 1 | C1 | 3668 | 3577 | 2.84% |
| Example 2 | C2 | 4580 | 4497 | 1.81% |
| Example 3 | C3 | 3859 | 3768 | 2.36% |
| Example 4 | C4 | 4207 | 4105 | 2.42% |
| Example 5 | C5 | 4746 | 4698 | 1.01% |
| Example 6 | C6 | 4983 | 4938 | 0.90% |
| Example 7 | C7 | 5012 | 4926 | 1.72% |

(continued)

| Examples | Gel name | Before sterilization | After sterilization | G' loss rate |
|---|---|---|---|---|
| | | G' (Pa) | G' (Pa) | |
| Comparative example 1 | B1 | 948 | 415 | 56.22% |
| Comparative example 2 | B2 | 997 | 432 | 56.67% |
| Comparative example 3 | B3 | 876 | 479 | 45.32% |
| Comparative example 4 | B4 | 785 | 399 | 49.17% |

Test example 2: Determination of SEM and pore diameter of gel

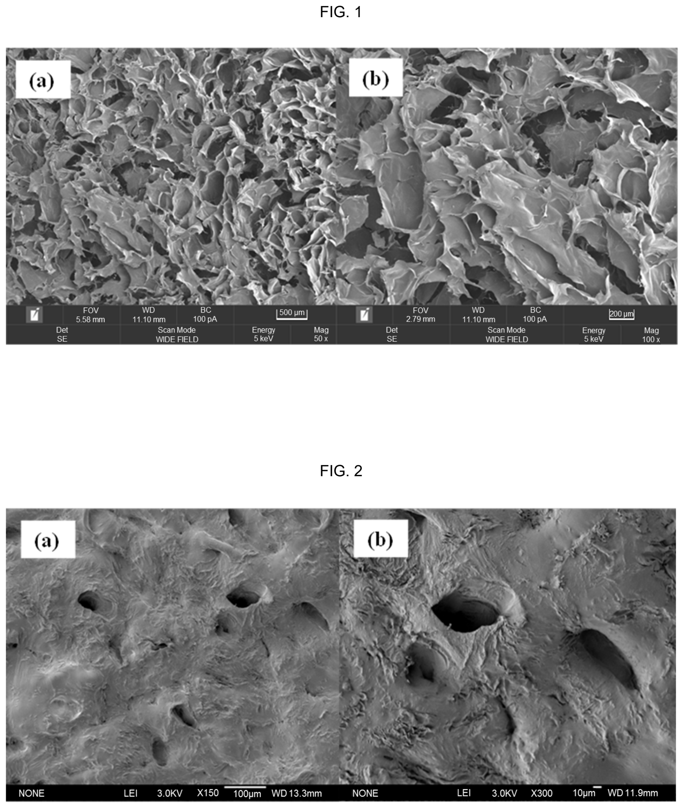

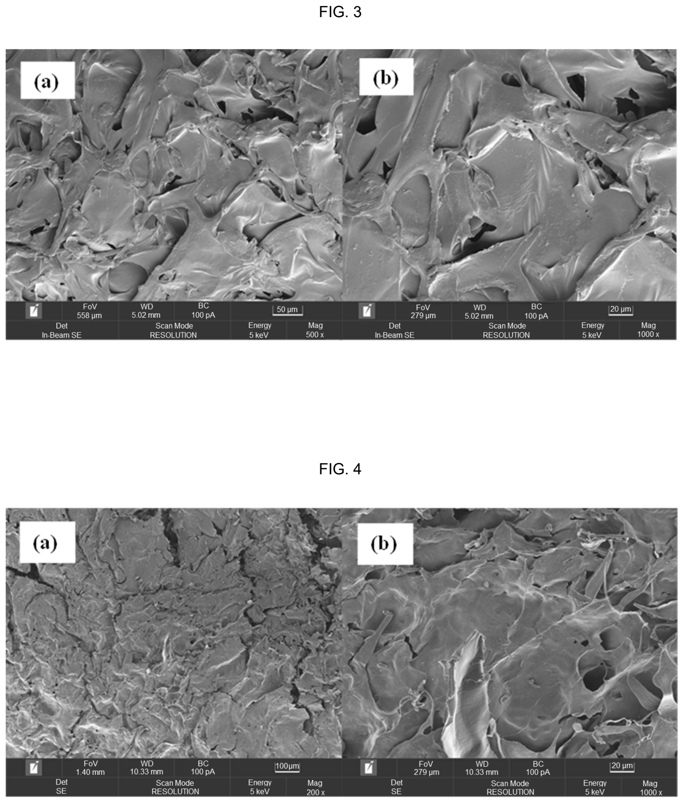

A pore structure of the gel was observed by a Scanning Electron Microscope. A freeze-dried cryogel sponge was cut into small blocks by a blade, the small blocks were fixed on an electron microscope stage by using a conductive adhesive and were subjected to metal spraying for 60 s, analysis was performed by using a field emission scanning electron microscope, SEM results for the cryogel sponges C6 and B1-B4 were shown in FIGs. 1-5, respectively, and FIG. b was a partial enlarged view of FIG. a.

The pore diameters of the gels were counted and analyzed by using ImageJ software and the SEM images, and Table 2 was a pore diameter distribution of the gels C1-C7 and B1-B4. From Table 2, it might be seen that, the pore diameters of the gels C1-C7 were all between 80 $\mu$m and 760 $\mu$m. By comparing the data of gels B1 and C6, as well as the gels B2 and C7, it might be found that the pore diameters of the B1-B2 that were not subjected to the pre-crosslinking treatment were very small, which were much less than the pore diameters of the C6 and C7, and it indicated that the pre-crosslinking process had a certain impact on the formation of macroporous structures of the gels, which might because the pre-crosslinking process might regulate and control the number of sites of the crosslinking reaction of the cryogels, such that the pore diameters of the gels were affected. Furthermore, by analyzing the results of the C1-C7, it was found that, when the pH value of the system was 4-5.3, the pore diameters of the prepared cryogels were 420-760 $\mu$m; when the pH of the reaction system was 5.4-6.2, the pore diameters of the prepared cryogels were 155-419 $\mu$m; and when the pH of the reaction system was 6.3-7.4, the pore diameters of the prepared cryogels were 80-154 $\mu$m. From FIGs. 1-5, it might further be seen that, the pore diameters of the cryogels B1-B4 were smaller, and the porosity was lower; and the cryogel C6 had an interconnecting macroporous structure with an irregular shape and high porosity, and thus had the potential to be applied to tissue engineering as a cellular scaffold, such that it also indicated that the formation of a porous structure was facilitated by performing the pre-crosslinking treatment before freezing. If the pre-crosslinking treatment was not performed or was not performed under appropriate conditions, later crosslinking efficiency was affected significantly, which was difficult to form the multi-site crosslinking, thus affecting the formation of the pore structures of the gels.

Table 2 Pore diameter distribution of gel

| Examples | Cryogel | Pore diameter ($\mu$m) |
|---|---|---|
| Example 1 | C1 | 650-760 |
| Example 2 | C2 | 420-510 |
| Example 3 | C3 | 190-269 |
| Example 4 | C4 | 155-189 |
| Example 5 | C5 | 511-645 |
| Example 6 | C6 | 270-419 |
| Example 7 | C7 | 80-154 |
| Comparative example 1 | B1 | 20-50 |
| Comparative example 2 | B2 | 10-50 |
| Comparative example 3 | B3 | 5-30 |
| Comparative example 4 | B4 | 5-10 |

Test example 3: Determination of porosity of gel

2 g of the gels C1-C7 and B1-B4 prepared in Examples 1-7 and Comparative examples 1-4 were each weighed and placed in a 500-mesh dry sieve, the mass $M_0$ of the dry sieve was weighed and recorded, a NaCl solution (0.9%) was added to the sieve to cause the gels to fully soak in the NaCl solution, and the gels were fully swelled overnight. The sieve was placed on a lens paper, excessive moisture was dried through wiping, and then the total mass of the swelled gels and the sieve were weighed and recorded as $M_1$. Then, the sieve with the gels was dried until it was completely dried, and the weight of the dried gels and the sieve were weighed and recorded as $M_2$. Finally, the porosity of the gels was calculated, and a calculation formula was shown as follows.

$$Gel\ porosity\ (\%) = (M_1 - M_2)/(M_1 - M_0) * 100\%$$

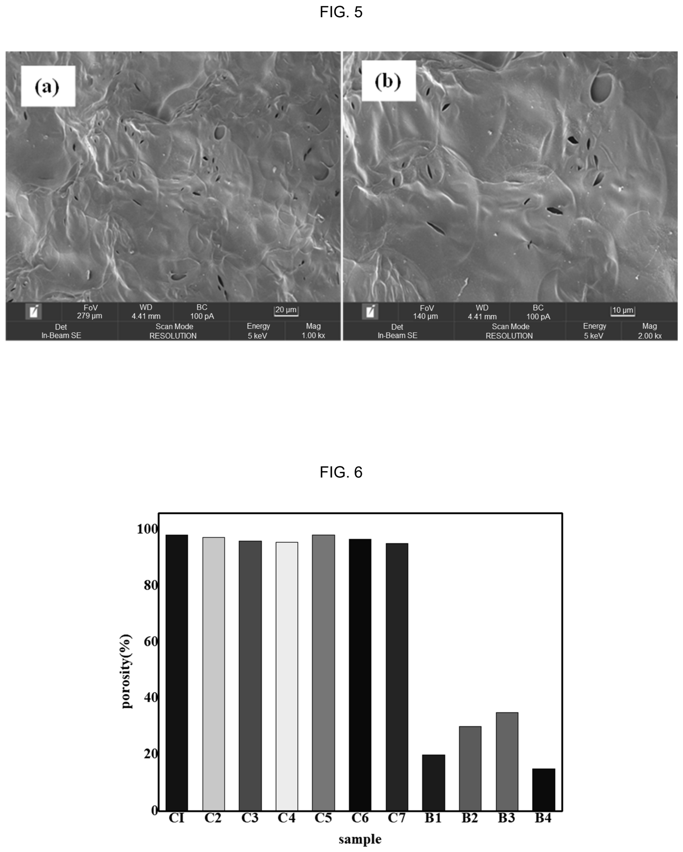

The determination results of the gel porosity were shown in FIG. 6. The porosity of the gels C1-C7 of the present disclosure was more than 95%, and the porosity of the gels B1-B4 obtained in Comparative examples 1-4 was 15%-35%.

Test example 4: Determination of swelling rate of gel

In order to evaluate the water absorption of the gel, the mass of the gels C1-C7 and B1-B4 after constant weight was recorded as W1, and then the gels were immersed in a phosphate buffer with pH = 7.0 to fully swell the gels. Samples were collected every 0.2 h, unabsorbed moisture was removed gently with a filter paper, and then weighing was performed. The test was stopped when the samples were swelled and reached a saturation point ($W_{hyd}$). The swelling rate of the gel was calculated by the following formula.

$$Swelling\ rate = (W_{hyd} - W_1)/W_1 \times 100\%$$

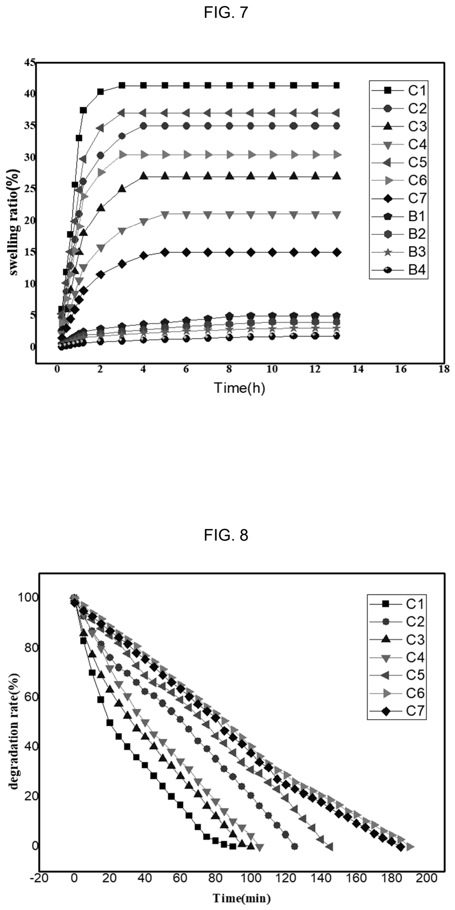

The test results for the swelling rates of the gels C1-C7 and B1-B4 were shown in FIG. 7. The swelling rates of the gels C1-C7 of the present disclosure were 15.00%-41.4%, and the swelling rates of the gels B1-B4 were only 1.8%-5.0%, where the gel C1 had the highest swelling rate and the fastest water absorption, and reached the saturation point earliest, which was because the gel C1 had a larger pore diameter and higher porosity, such that more moisture might be absorbed more quickly, thereby reaching the saturation point of water absorption more earlier. The swelling rates of the gels B1-B4 obtained in the comparative examples were all relatively low, which was because the gels had smaller pore diameters and lower porosity, such that a water absorption rate was slower, and more time was required to reach the saturation point. By using the high water absorption of the cryogel, the cryogel might be used as a hemostatic material to be applied to tissue engineering.

Test example 5: Determination of in vitro degradation performance of gel

A 10 mL glass bottle was taken, 1.0 mL of a 75 U/mL enzyme solution was added, 2.0 mL of a gel sample was pushed without needles, then mixing was performed for 60 s in a vortex mixer, ensuring that the gel sample and the enzyme solution were uniformly mixed, dynamic viscosity ($\eta$, Pa·s) was determined at 37 °C by using a rheometer: loading gap: 1000 $\mu$m, test mode: flow peak hold mode; and test time: 14400 s.

FIG. 8 showed a degradation curve of the gels C1-C7. From the curve, it might be learned that, the stability of the gels was high, and degradation times were all over 90 min; in the polyglutamic acid gels C1-C6, the elastic modulus of the C6 after sterilization was the highest, and the degradation time was the longest, which might reach 190 min. It might be seen that, the degradation time of the gel was basically proportional to the elastic modulus thereof, that is, if the elastic modulus of the gel was greater, it was more difficult to degrade, and the degradation time was longer.

Test example 6: Determination of mechanical property of gel

A proper amount of the gel (a cylinder with a direct diameter being 10 mm and a height being about 10 mm) was taken and subjected to uniaxial stretching on a universal electronic testing machine to test its mechanical property, a 1000 N tension sensor was selected, a stretching rate was 120 mm/min, and a zero clearing operation was required before each stretching. After the experiment was completed, a force-displacement curve of the gel sample could be obtained, and stress and strain were calculated according to the following formula, thereby obtaining a stress-strain curve.

Stress:

$$\text{Stress} = \text{Force/Area}$$

Strain:

$$\text{Strain} = \text{Displacement/10*100\%}$$

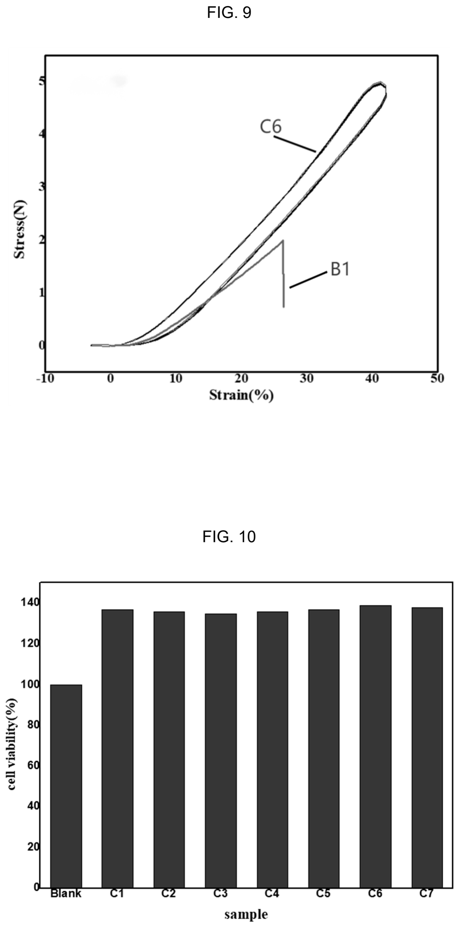

The mechanical property of the gel was determined according to the above test method. FIG. 9 showed a stress-strain curve of the gels C6 and B1. The shape of the gel C6 remained intact after 10 cycles of compression, and the stress was about 5 N when the strain was 42%; and the stress of the gel B1 was about 1.99 N when the strain was 26%, and in this case, the gel no longer held its shape and broke. The reason might be that, the structure of the gel obtained through the pre-crosslinking and freezing-thawing crosslinking processes was the four-site crosslinking, such that the gel had a more compact structure, higher stability, and stronger mechanical property under pressure; and the gel B1 was double-site crosslinking, and its skeleton was not compact enough, such that the structure was not stable, and the mechanical property was poor during pressure process.

Test example 7: Impact of gel on cell proliferation

L-929 cells were cultured by placing in an MEM culture medium containing antibiotic (100 U/mL penicillin and 100 ug/mL streptomycin) and 10% serum at 37 °C and under a 5% $CO_2$ saturated humidity condition. A sterile operation was used when the cells were cultured. The cells were digested by using trypsin when growing to convergence, the cells were collected, and the following test was performed by regulating a cell concentration to $1 \times 10^5$ cell/mL.

The above cell suspension was added to a 96-well plate, with 100 $\mu$L in each well, there were $1 \times 10$" cell/well in total, and culture was performed for 24 hours at 37 °C and 5%$CO_2$. After the end of culture, the culture medium in a culture plate was discarded, and extract liquid of the gels C1-C7 and a blank control were added in groups of 6 wells each, and placed in a 37 °C and 5% $CO_2$ saturated humidity incubator for culture. 1 mL of a trypsin solution containing 0.1% EDTA was added to the L-929 cell culture medium, the L929 cells were inoculated in a 96-well plate according to $1 \times 10^4$ cell/well, with 100 $\mu$L solution in each well, and the well plate was continuously placed in the cell incubator to promote cell growth. Comparative cell proliferation experiments with blanks were performed synchronously.

A cell proliferation rate of the gel was determined by an MTT method. After 24 hours of cultivation respectively, 100 $\mu$L of an MTT aqueous solution (with the concentration being 5 mg·mL-1) was added to each well, and placed in the incubator for continuous cultivation for 4 hours. Then the MTT solution was removed, 150 $\mu$L of dimethyl sulfoxide was added to dissolve formazan crystals, and the absorbance of the solution was detected at a wave length of 570 nm by using a microplate reader, so as to determine a cell proliferation degree of the gel to the L-929. The cell proliferation rate was calculated according to the following formula.

$$\text{Cell proliferation rate (\%)} = (As/Ac) \times 100\%$$

As was the absorbance of a sample solution at 570nm, and $A_c$ was the absorbance of a blank control at 570nm.

Cell proliferation results were shown in FIG. 10. From the figure, it might be seen that the cell viability of the gels C1-C7 of the present disclosure at 24 hours was 137%, 136%, 135%, 136%, 137%, 139%, 138%, respectively, which were all significantly higher than the 100% cell viability of a comparative blank experiment without adding gels. From the cell viability, it might be seen that, the gel of the present disclosure had no cytotoxicity, and the porous structure of the gel had a promotion effect on cell growth to a certain extent.

Test example 8: Detection of remaining active sites in multi-site crosslinked gels such as double-site and three-site or four-site

Approximately 2 g of ninhydrin hydrate was taken, 100 mL of the purified water was added to dissolve the ninhydrin hydrate, and well mixed; and the mixture was preserved under dark at 2-8 °C for later use. 54.6 g of sodium acetate was taken, 20 mL of a 1 mol/L acetic acid solution was added to dissolve the sodium acetate, and water was added to dilute the mixture to 500 mL for later use. A spermidine standard and a spermine standard were respectively taken, placed in a 10 mL measuring bottle, diluted to scale with the purified water, and well shaken, so as to prepare a 500 $\mu$g/mL spermidine standard solution and a 500 $\mu$g/mL spermine standard solution. When being used, the solutions were quantitatively diluted to 10, 50, 100, 200 $\mu$g/mL, and an original 500 $\mu$g/mL standard solution was used as a standard curve.

The cryogel in Examples 1-7 and Comparative examples 1-4 before moist heat sterilization and the above spermine standard solutions and spermidine series standard solutions were taken, 1 mL of each of the above was taken, 2.0 mL of an acetic acid-sodium acetate buffer solution and 2.0 mL of the ninhydrin hydrate solution were added in sequence, plugs were added, after well mixing, the mixture was placed in a 70 °C water bath for heating for 30 min, taken out and immediately cooled to room temperature, diluted to 25 mL with the purified water, and well mixed; the mixed solution was taken, the absorbance of an amino group derivative at the wave length of 565 nm was determined, and the absorbance of an imino group derivative at the wave length of 400 nm was detected at the same time; and blank correction was performed with the purified water by using the same method.

The residue proportion (%) of amino or imino was calculated according to the following formula.

$$\text{Residue proportion} = (A_0 - A_i)/A0 \times 100\%$$

In the formula, $A_0$ was an absorbance value obtained by measuring a control solution, and $A_i$ was an absorbance value obtained by measuring a sample.

One spermidine molecule contained one imino group and two amino groups, and one spermine molecule contained two imino groups and two amino groups.

Test results were shown in Table 3.

Table 3 Residue Proportion of amino or imino in gel

| Examples | Cryogel | Amino residues | Imino residues |
|---|---|---|---|
| Example 1 | C1 | 9.07% | 9.71% |
| Example 2 | C2 | 8.81% | 9.02% |
| Example 3 | C3 | 9.13% | 9.85% |
| Example 4 | C4 | 8.93% | 9.35% |
| Example 5 | C5 | 8.16% | 9.21% |
| Example 6 | C6 | 6.35% | 7.21% |
| Example 7 | C7 | 7.41% | 8.31% |
| Comparative example 1 | B1 | 14.25% | 87.13% |
| Comparative example 2 | B2 | 13.35% | 88.31% |
| Comparative example 3 | B3 | 23.41% | 91.41% |
| Comparative example 4 | B4 | 27.34% | 90.56% |

From the results of Table 3, it might be learned that, the gels C1-C7 obtained in Examples 1-7 were multi-site crosslinking such as three-site or four-site, and amino and imino residues of the spermidine or spermine were all lower than 10%, indicating that the reaction efficiency of the amino and imino reached more than 90%. Therefore, it indicated that through the pre-crosslinking and freezing-thawing crosslinking processes, three-site crosslinking occurred in the spermidine and the polyamino acid, and four-site crosslinking occurred in the spermine and the polyamino acid, thereby achieving the efficient utilization of the crosslinking agent, which was of great significance for rationally controlling the residual amount of the crosslinking agent and for improving the safety of the clinical use of the product, thereby further confirming the validity of the multi-site crosslinking such as three-site or four-site occurred between the crosslinking agent and the polyamino acid in the present disclosure. In Comparative examples 1-2, due to the lack of the pre-crosslinking process, a main reaction group thereof was the amino, the reaction efficiency was about 85%, and the imino residues in the spermidine or spermine reached up to 87% or more, indicating that there were fewer imino groups participating in the reaction, which might because the pre-crosslinking treatment was not performed before freezing, there was a large distance between spermine or spermidine molecules and the molecular chain of the polyamino acid that had been fixed by the ice crystals after freezing, the imino group with low crosslinking reaction activity had an extremely-low collision probability with the carboxyl of the molecular chain of the polyamino acid substance, leading to a reduction in efficiency during the crosslinking process. Therefore, the crosslinking reaction was mainly that the amino groups on two ends of the crosslinking agent participated in the reaction to form the double-site crosslinking, which was difficult to form the multi-site crosslinking such as three-site or four-site with a more stable structure. Compared with Example 6, the amino and imino residues of the gels obtained in Comparative examples 3-4 were much higher than those in Example 6, further indicating that if the pre-crosslinking treatment was not performed under appropriate conditions, the crosslinking efficiency of the subsequent freezing process was greatly reduced, and what mainly occurred was also a double-site crosslinking reaction involving amino groups.

Finally, it should be noted that, the above embodiments are merely for describing and not intended to limit the technical solutions of the present disclosure. Although the present disclosure has been described in detail with reference to the above embodiments, those of ordinary skill in the art should understand that, they can still make modifications to the technical solutions recited in the above embodiments or make equivalent replacements to a part or all of the technical features thereof; and the modifications or replacements do not cause essence of the corresponding technical solutions to depart from the scope of the technical solutions of the embodiments of the present disclosure.

**Claims**

1. A polyamino acid gel material, wherein the material is obtained by performing pre-crosslinking and freezing-thawing treatments on endogenous polyamine and polyamino acid under the action of an activating agent, wherein the crosslinking of the endogenous polyamine and the polyamino acid comprises three-site crosslinking or four-site crosslinking.

2. The gel material according to claim 1, wherein the endogenous polyamine comprises spermidine and/or spermine.

3. The gel material according to claim 1, wherein the polyamino acid is polyglutamic acid and/or polyaspartic acid.

4. The gel material according to claim 1, wherein a pore diameter of the gel material is 80-760 $\mu$m; and/or the porosity of the gel material is more than 95%, preferably, more than 98%.

5. The gel material according to claim 1, wherein the activating agent comprises one or more of water-soluble carbodiimide, a carbonium salt, and 4-(4,6-dimethoxy-1,3,5-triazin-2-yl)-4-methyl morpholinium chloride.

6. A method for preparing a polyamino acid gel material, wherein polyamino acid and endogenous polyamine are mixed to obtain a mixed solution, a pH of the mixed solution is regulated to 4.00-7.40, an activating agent is added, pre-crosslinking is performed for 5-60 minutes at 25-60 °C after stirring, then freeze-crosslinking is performed, a product after freeze-crosslinking is thawed, and then a freezing-thawing process is repeated for 0-3 times, so as to obtain a gel material.

7. The preparation method according to claim 6, wherein a temperature for freeze-crosslinking is -80°C to -10 °C, and a time is 2-48 h; and a temperature for thawing is 10-60 °C, and a thawing time is 0.5-8h.

8. The method according to claim 6, wherein the endogenous polyamine comprises spermine and/or spermidine;

   preferably, the activating agent comprises one or more of water-soluble carbodiimide, a carbonium salt, and 4-(4,6-dimethoxy-1,3,5-triazin-2-yl)-4-methyl morpholinium chloride;
   preferably, the polyamino acid comprises polyglutamic acid and/or polyaspartic acid; and/or
   a solvent of the mixed solution is selected from the mixing of one or more of water, soluble alcohol, soluble ketone, DMF, DMA, and DMSO; and preferably, the solvent is water.

9. The method according to claim 6, wherein a mass concentration of the polyamino acid in the mixed solution is 10-160 mg/mL;

   when the endogenous polyamine is spermine, the spermine is added in an amount of 1.0-62% of a molar weight of a polyamino acid structure unit; and
   when the endogenous polyamine is spermidine, the spermidine is added in an amount of 0.5-52% of a molar weight of a polyamino acid structure unit.

10. The method according to claim 9, wherein the activating agent is added in an amount of 0.5-550% of the molar weight of the polyamino acid structure unit, preferably 50-500%, and more preferably 50-300%; when the activating agent is water-soluble carbodiimide, the method further comprises adding an auxiliary agent to the mixed solution;

    the auxiliary agent comprises any one or more of N-hydroxysuccinimide, sulfonated N-hydroxysuccinimide, tert-butanol, and 1-hydroxybenzotriazole; and

preferably, the auxiliary agent is added in an amount of 10-50% of the mass of the carbodiimide.

11. The method according to claim 6, wherein the method further comprising adding the thawed product to a phosphate buffer with a concentration of 10-25 mg/mL, and performing moist heat sterilization to obtain a gel material.

12. A gel material prepared by the method according to any one of claims 6 to 11.

13. An application of the gel material according to any one of claims 1 to 5, a gel material prepared by the method according to any one of claims 6 to 11, or the gel material according to claim 12 in preparation of medical, beauty, and health care products, wherein
the medical and beauty products comprise: one or more of soft tissue filling materials, cartilage repair materials, tissue implant materials, coatings of biomaterial implants, tissue engineering scaffold materials, drug sustained-release media, drug targeting carriers, and wound dressings.

FIG. 1

FIG. 2

FIG. 3

FIG. 4

FIG. 5

FIG. 6

FIG. 7

FIG. 8

FIG. 9

FIG. 10

## INTERNATIONAL SEARCH REPORT

| International application No. |
| --- |
| **PCT/CN2022/143868** |

| A. | CLASSIFICATION OF SUBJECT MATTER |
| --- | --- |

C08J 3/24(2006.01)i; C08J 3/075(2006.01)i; C08K5/17(2006.01)i; A61L27/52(2006.01)i

According to International Patent Classification (IPC) or to both national classification and IPC

| B. | FIELDS SEARCHED |
| --- | --- |

Minimum documentation searched (classification system followed by classification symbols)

IPC: C08J C08K A61L

Documentation searched other than minimum documentation to the extent that such documents are included in the fields searched

Electronic data base consulted during the international search (name of data base and, where practicable, search terms used)

CNABS: CNTXT; CNKI; VEN; ENTXT; Web of Science: 爱美客, 温慧芳, 李睿智, 张堃, 谷诗伟, 医用材料, 凝胶, 水凝胶, 氨基酸, 谷氨酸, 天冬氨酸, 交联, 精胺, 精素, 三元胺, 四元胺, 三氨基, 四氨基, 冷冻, 解冻, 冻融, 活化, 催化, 孔隙, 假体, 缓释, 载体, hydrogel?, gel?, colloid?, Amino acid, Glutamic acid, Aspartic acid, PGA, PASP, Spermine, Spermidine, Triamine, Tetraamine, Triamino, Tetraamino, Freez+, thaw+, EDC, DMTMM, Porosity

| C. | DOCUMENTS CONSIDERED TO BE RELEVANT |
| --- | --- |

| Category* | Citation of document, with indication, where appropriate, of the relevant passages | Relevant to claim No. |
| --- | --- | --- |
| Y | Tama's Gyenes et al. "Synthesis and swelling properties of novel pH-sensitive poly(aspartic acid) gels"<br>*Acta Biomaterialia*, 01 January 2008 (2008-01-01), pages 733-744<br>ISSN: 1742-7061,<br>page 733, abstract, and page 736, section 3.3.1 | 1-13 |
| Y | ZHAO, Ying et al. "Poly(aspartic acid) Super-Absorbent Resin Produced by Chemical Crosslinking and Physical Freeze/Thawing"<br>*Macromolecular Chemistry and Physics*, Vol. 207, No. 14, 14 July 2006 (2006-07-14), pages 1297-1305<br>ISSN: 1521-3935,<br>page 1298, left side, line 19 to page 1304, right side, line 29 | 1-13 |
| A | CN 113896915 A (IMEIK TECHNOLOGY DEVELOPMENT CO., LTD.) 07 January 2022 (2022-01-07)<br>entire document | 1-13 |

| ✓ Further documents are listed in the continuation of Box C. | ✓ See patent family annex. |
| --- | --- |

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| **04 August 2023** | **09 August 2023** |

| Name and mailing address of the ISA/CN | Authorized officer |
| --- | --- |
| **China National Intellectual Property Administration (ISA/CN)**<br>**China No. 6, Xitucheng Road, Jimenqiao, Haidian District, Beijing 100088** | |
| | Telephone No. |

Form PCT/ISA/210 (second sheet) (July 2022)

## INTERNATIONAL SEARCH REPORT

International application No.

**PCT/CN2022/143868**

### C.    DOCUMENTS CONSIDERED TO BE RELEVANT

| Category* | Citation of document, with indication, where appropriate, of the relevant passages | Relevant to claim No. |
|---|---|---|
| A | CN 102321256 A (NANKAI UNIVERSITY) 18 January 2012 (2012-01-18)<br>entire document | 1-13 |
| A | CN 103157129 A (SHANGHAI UNIVERSITY) 19 June 2013 (2013-06-19)<br>entire document | 1-13 |

Form PCT/ISA/210 (second sheet) (July 2022)

**INTERNATIONAL SEARCH REPORT**
Information on patent family members

International application No.

**PCT/CN2022/143868**

| Patent document cited in search report | | | Publication date (day/month/year) | Patent family member(s) | | | Publication date (day/month/year) |
|---|---|---|---|---|---|---|---|
| CN | 113896915 | A | 07 January 2022 | None | | | |
| CN | 102321256 | A | 18 January 2012 | CN | 102321256 | B | 29 August 2012 |
| CN | 103157129 | A | 19 June 2013 | CN | 103157129 | B | 01 October 2014 |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **MADEO et al.** *Science*, 2018, vol. 359, 410